# EUROPEAN PATENT APPLICATION

(11) **EP 0 574 091 A2**
(43) Date of publication of application: **15.12.1993**
(21) Application number: 93201676.9
(22) Date of filing: 11.06.1993
(51) Int. Cl.: A61C 8/00, A61F 2/28, A61B 17/58, A61B 17/00

(54) **Bioresorbable barrier element**

(30) Priority: 11.06.1992 NL 9201037; 11.11.1992 NL 9201973
(71) Applicant: DENTAL INNOVATIONS B.V., NL-9951 BA Winsum (NL)
(72) Inventor: DENTAL INNOVATIONS B.V., NL-9951 BA Winsum (NL)
(74) Representative: Smulders, Theodorus A.H.J., Ir.

(57) **Abstract**

The barrier element according to the invention comprises a rigid screening portion (1) formed from synthetic material. This renders the barrier element easy to implant and resistant to mechanical loads, and it affords greater freedom in choosing the shape of the space to be screened. Preferably, at least one flexible edge (2) extends along the screening portion (1) for obtaining a reliable seal along the edge of the barrier element.

## Description

This invention relates to a barrier element according to the preamble of claim 1.

Such barrier elements are known from a publication by Caton *et al*, titled "Synthetic Bioabsorbable Barrier for Regeneration in Human Periodontal Defects" in the Journal of Dental Research 69: Special Issue, 1990, Abstract # 1336.

These known barrier elements are constructed as tissues of bioresorbable fibrous material. An advantage of these known barrier elements is that they need not be removed anymore once they have been fitted. Such an additional operation entails renewed damage to the tissue of the patient, constitutes a psychological burden on the patient and moreover costs additional time and money.

However, a drawback of these known barrier elements is that they are difficult to mount and the range of application is limited because the tissue must be stretched over the space to the screened. In the case of their application for the regeneration of supporting tissue of dental elements or dental implants, this means, for instance, that the use of such barrier elements remains out of reach of most dentists who have a general practice.

A further drawback of these known barrier elements is that they exhibit poor stability in mounted condition. As a result, there is a relatively great risk of the space where osteogenesis is intended not being screened off to a sufficient extent. In dental surgery applications, it appears that in many cases these barrier elements are deformed after implantation, *inter alia* through tissue pressure and possibly through forces exerted during chewing.

Further known are barrier elements made from PFTE (sometimes designated by the brand name of Teflon), which are marketed under the name of "GORETEX Periodontal Material". These barrier elements are to be removed after the patient is cured. Further, practically speaking, these barrier elements have substantially the same mechanical properties as the barrier elements described above. Accordingly, similar drawbacks are associated with them.

The object of the present invention is to overcome these drawbacks by providing a barrier element which is easy to fit and provides a reliable screen for the space where osteogenesis is intended.

This object is realized according to the present invention by using the characterizing features according to claim 1 in a barrier element of the type described in the preamble.

Because the barrier element according to the invention comprises a bending-stiff screening portion formed from synthetic material, it need not be stretched when implanted, but it can simply be arranged so that the screening portion extends along the space to be screened, the shape of the screening portion determining the shape of the space where osteogenesis will occur. Owing to the bending stiffness of the screening portion, the barrier element according to the invention exhibits better resistance to mechanical loads, so that the free space where osteogenesis is intended is screened off more reliably.

A further advantage of the barrier element according to the present invention is that it provides greater freedom as regards the shape of the space where osteogenesis is allowed to occur.

A still further advantage of the barrier element according to the invention is that it can also serve as a supporting element for maintaining the relative position of portions of bone tissue that are to grow together. The barrier element according to the invention thus provides the possibility of allowing portions of bone tissue with bone tissue lost between them to grow together again, without transplantating bone tissue in replacement of the bone tissue lost.

The use of the bending-stiff barrier elements according to the invention may lead to these elements being made of relatively heavy dimensions in order to achieve the desired bending stiffness. In that case, to prevent foreign-body reactions, it is particularly advantageous if the barrier element has a composite structure with a first portion made from a first resorbable material and a second portion made from a second resorbable material, these materials having a structure and composition such that, after implantation, the material of the first portion on average degrades more slowly than the material of the second portion.

The invention may further be embodied by an assembly of parts according to claim 31, which further comprises fastening means for attaching the barrier element according to the invention to bone tissue quickly and easily. The hold-down edge of the head will crease and be upset against portions of the barrier element that adjoin material of the barrier element that has been pressed against bone tissue by the head. Because the fastening elements can be fitted by exerting an exclusively axial pressure, the shank need not take up any torsional load resulting from forces being exerted as the fastening element is being turned into the supporting surface and it may be relatively thin. This, in turn, means that the concentrations of foreign material in the patient's body are limited, so that foreign-body reactions are limited.

The invention may further be embodied by a mandrel according to claim 32 or 33 for rolling a barrier element according to the invention into a tube, a method according to claim 34 for making a barrier element according to the invention and an injection moulding machine according to claim 35 for practising the method according to claim 34.

Hereinafter, the invention will be further explained on the basis of some exemplary embodiments, with reference to the accompanying drawings, which are all diagrammatic representations.
Fig. 1 is a sectional view taken on the line II-II in Fig. 2 of an exemplary embodiment of the invention;
Fig. 2 is a top plan view of the barrier element shown in Fig. 1;
Fig. 3 is a similar view to Fig. 1, of a further exemplary embodiment of the invention;

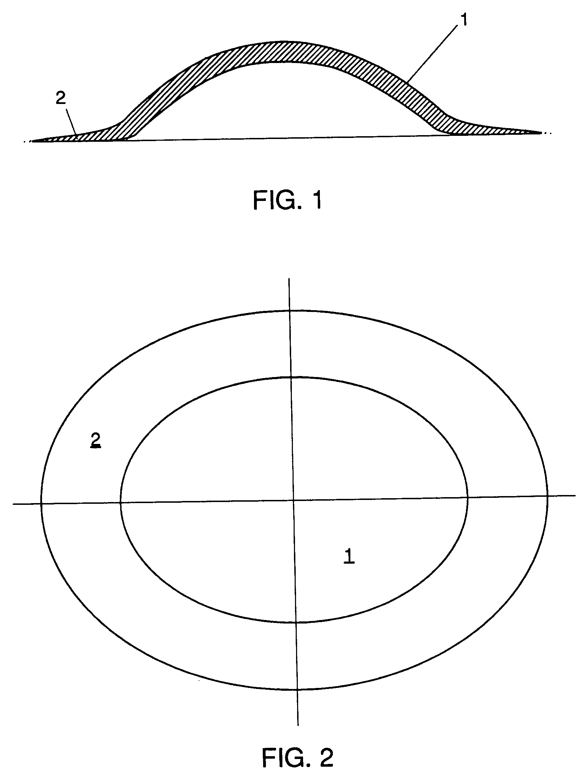

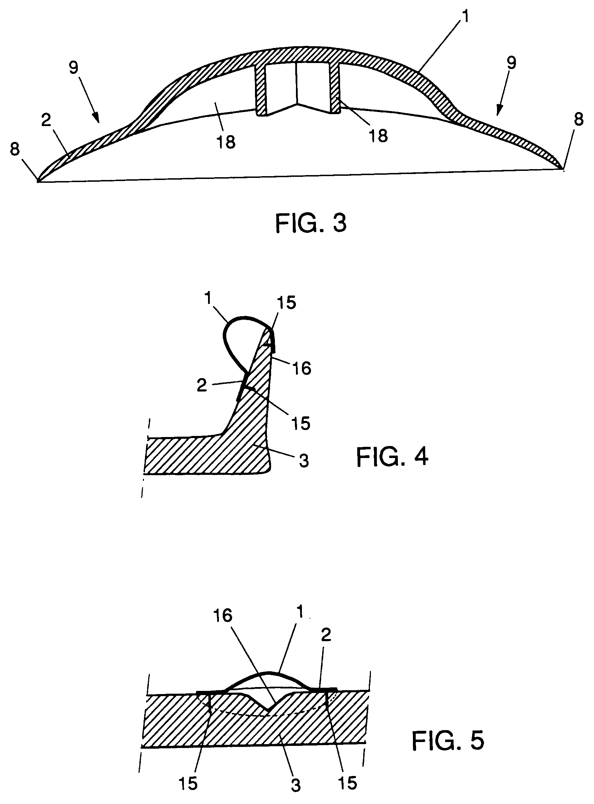

Figs. 3-6 are sectional views of examples of applications of the barrier elements shown in Figs. 1-3;

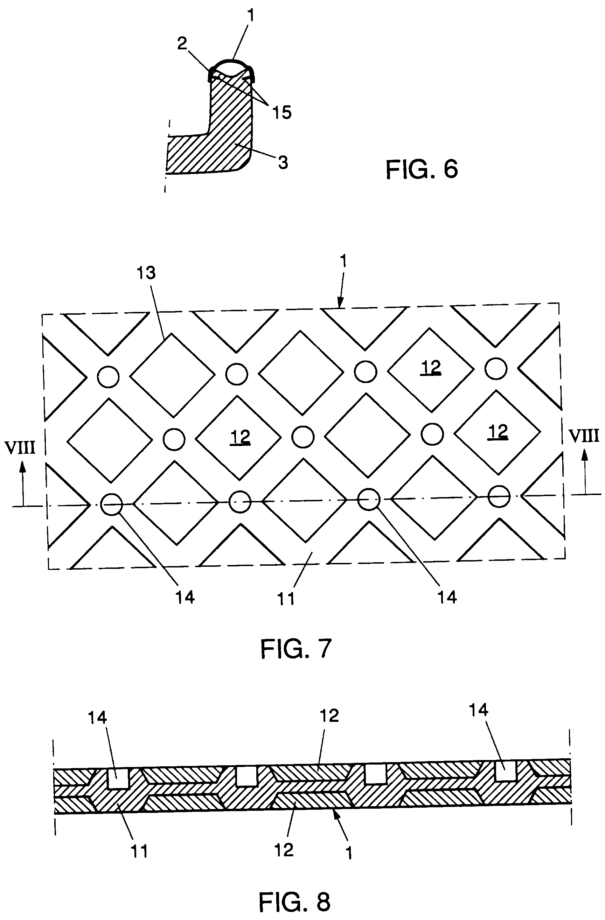

Fig. 7 is a front view of a further exemplary embodiment of the invention;
Fig. 8 is a sectional view taken on the line VIII-VIII in Fig. 7;

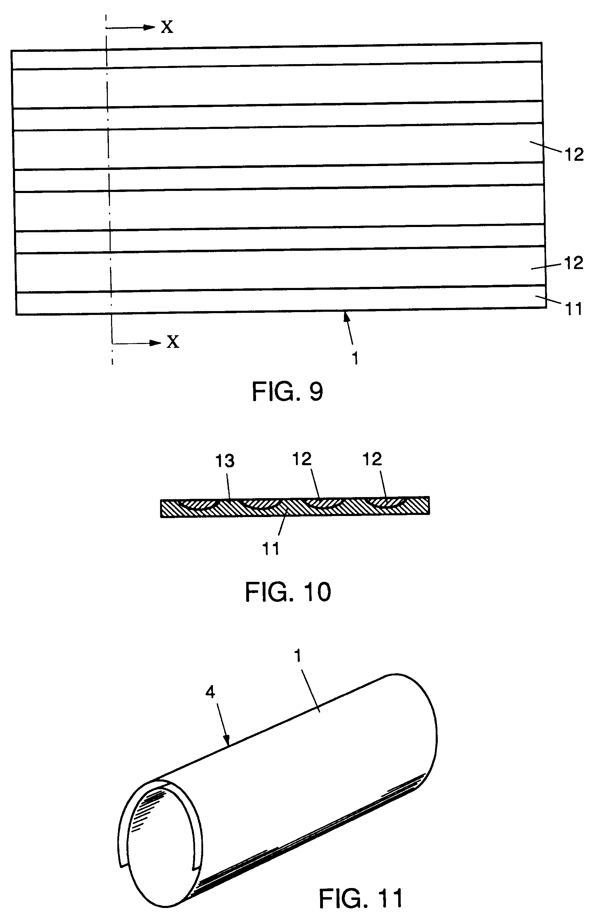

Fig. 9 is a view similar to Fig. 7, of a further exemplary embodiment of the invention;
Fig. 10 is a sectional view taken on the line X-X in Fig. 9;

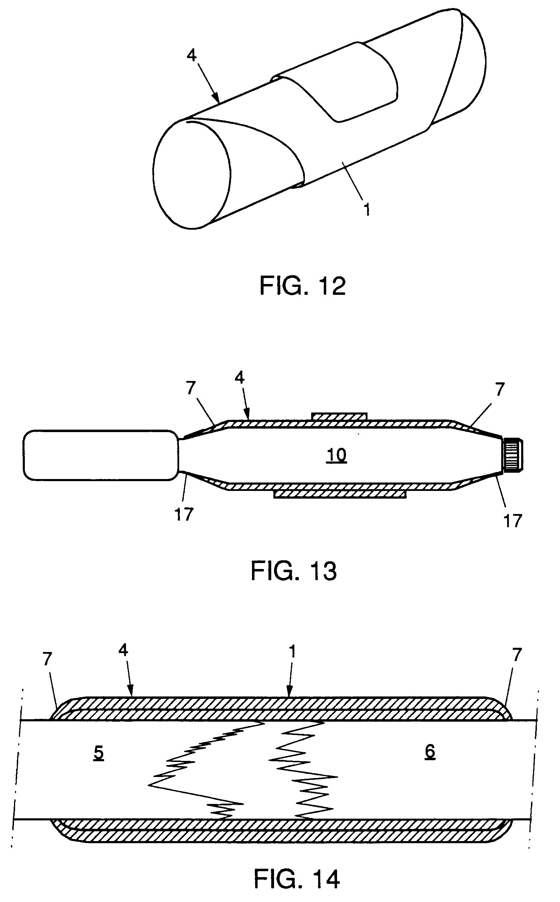

Figs. 11 and 12 are perspective views of barrier elements each rolled into a tube;
Fig. 13 is a cutaway view of a mandrel around which a barrier element as shown in Fig. 12 has been wound;
Fig. 14 is a cutaway view of an example of an application of a barrier element rolled up into a tube;

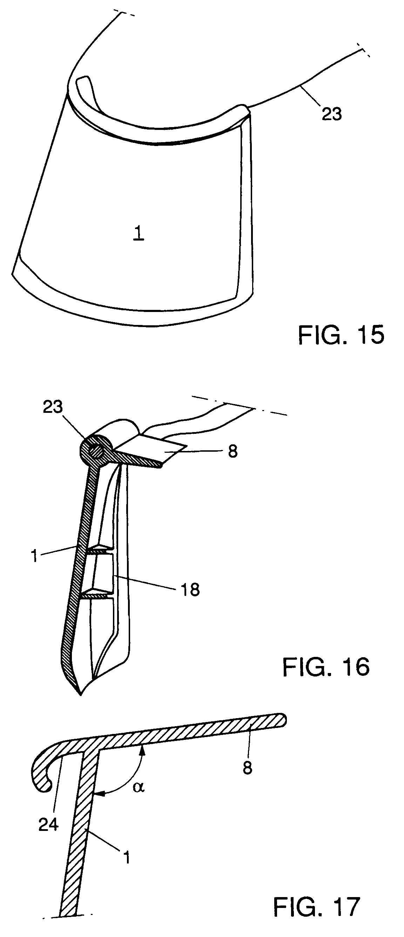

Fig. 15 is a perspective view of a barrier element for arrangement against a dental element;
Fig. 16 is a perspective view of a cutoff portion of a barrier element according to the exemplary embodiment shown in Fig. 15;
Fig. 17 is a profile of a sealing edge to be fitted to a dental element for use in a barrier element as shown in Fig. 15;

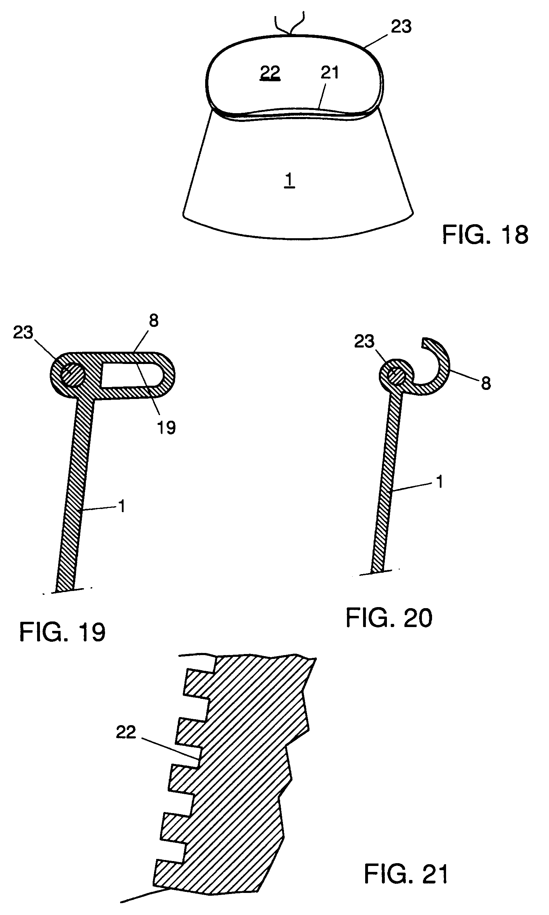

Fig. 18 is a schematic top plan view of a dental element having a barrier element according to Figs. 15 and 16 resting against it;
Figs. 19 and 20 are further profiles of sealing edges to be fitted to a dental element, for use in a barrier element according to Fig. 15;

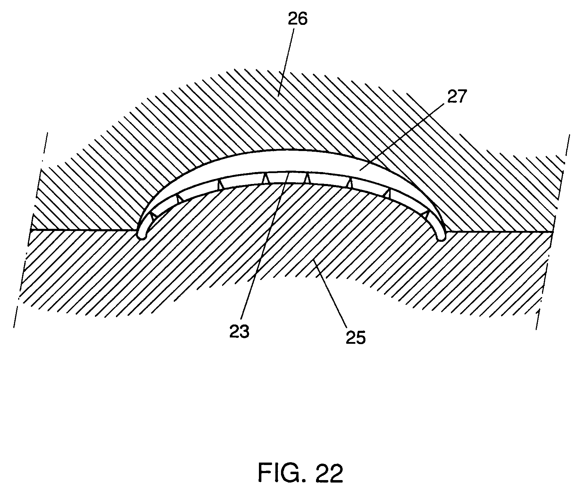

Fig. 21 is a greatly magnified view of an example of a surface texture of a barrier element according to the invention; and Fig. 22 is a schematic sectional view of a die designed for injection moulding around a stitching wire.

The barrier elements shown each comprise a form-retaining screening portion 1, which offers resistance against random deformations and, at body temperature, takes up its original shape after deformation (that is, of course, as long as the deformation does not exceed given maximum deformations).

The known barrier elements consist of woven material or of material of paper-like properties as far as mechanical stability is concerned. These barrier elements are not bending-stiff and are to be stretched over the space to be screened. With the barrier element according to the invention, the shape of the screen is fixed when the barrier element is being formed, so that it need not be stretched when being implanted but can simply be fitted over the space to be screened by attachment to the adjacent bone tissue and, for dental applications, optionally to a dental element as well. Owing to the form-retaining property of the screen 1, the barrier element according to the invention exhibits better resistance to mechanical loads than a known barrier element stretched over a space. Thus, the space where osteogenesis is intended, is screened off more reliably.

The barrier element according to the invention can be designed in a large variety of shapes and dimensions, which affords greater freedom with regard to the shape and the space where osteogenesis is allowed to occur. A particularly good form-retaining ability can be obtained if the screening portion 1 is doubly curved, i.e., dish-shaped.

Further, the barrier element according to the invention can also serve as a supporting element for maintaining the relative position of portions of bone tissue which are to grow together, which makes it possible to allow portions of bone tissue with bone tissue lost between them to regenerate. Thus, the replacement of the lost bone tissue by transplantation of bone tissue can be dispensed with. Examples of barrier elements suitable for such applications are shown in Figs. 7-14.

The synthetic material is preferably a polylactic acid or a copolymer of lactic acid and glycolide. Such polymers are commercially available under the brand name PURASORB from Purac Biochem. b.v. at Gorinchem, the Netherlands.

In order to obtain the highest possible form-retaining ability, the screening portions 1 comprise a wall of substantially solid material. This wall may be composed of a plurality of different materials and may also be slightly permeable.

The barrier element according to the invention can be made in an efficient manner by injection moulding.

To increase the bending stiffness of the screening portion 1, this portion may be provided with ribs 18 or 19, as shown in Figs. 3 and 16.

The stiffening ribs 18 shown in Fig. 16 are designed as partitions whose height profile is such that, in implanted condition, they rest against a part of a dental element. Thus, even if very slight wall thicknesses are employed, a particularly robust screening is obtained, whilst the partitions do not eventually hamper the formation of bone because they are degraded over time, as are the other parts of the barrier element. If necessary, the height profile of the ribs 18 can be simply adapted, through cutting, to the corresponding shape of the dental element against which the barrier element is to rest. The fact that only a minor wall thickness is necessary provides the advantage that the total amount of material of a barrier element to be degraded and cleared by the body can be minimized and, moreover, is better distributed because it is not concentrated in one wall.

For dental applications in particular, the barrier element is preferably provided with a texture of grooves located next to each other, of a width and a depth of approximately 100 µm. An example of a greatly magnified sectional view of such a texture is depicted in Fig. 21. By virtue of such a texture, the growth of cells of the mucous membrane along the barrier element in a direction transverse to the grooves is retarded. Excessive growth of mucous membrane cells along the barrier element results in the formation of large pockets behind the gums at the location of the barrier element. As a result, the bone tissue grown in the space behind the barrier element will soon be attacked again.

The above-described texture, whose individual grooves can hardly be seen with the naked eye, if at all, can be formed in an injection-moulded product during the manufacture thereof. For that purpose, the texture must be provided in the die, which can be effected through electro-corrosive removal.

The exemplary embodiments shown in Figs. 1-6 each have a central, dish-shaped screening portion 1 and a thin-walled, annular portion 2 extending along the edge of the dish-shaped portion 1. The dish-shaped screening portion 1 screens a space where ostengenesis can take place and the thin-walled portion 2 around the screening portion 1 forms a flexible flange by which the barrier element can be reliably attached to a piece of bone tissue 3 (Figs. 4-6).

In the barrier element shown in Fig. 3, the ribs 19 extend radially from the centre of the screening portion 1 to the outer circumference thereof.

The thin-walled portion preferably has a thickness that decreases starting from the screening part 1, so that it is relatively stiff in the area of the screening portion and constitutes a supporting surface for transmitting forces exerted on the screening portion 1 to the adjacent bone tissue.

Fig. 4 shows a human lower jawbone 3, the upper part of which has become too narrow for a dental implant to be attached to it. A barrier element according to the invention is attached, adjacent the upper edge of the jawbone, to the inside of the jawbone by means of fastening means 15 (to be discussed hereinafter). In the space bounded by the screen 1, the jawbone will grow and widen, so that a dental implant can subsequently be reliably attached.

Figs. 5 and 6 show a barrier element according to the invention fitted over a concavity 16 formed in a jawbone. The flexible edge is secured to the bone 3 by means of fastening means 15.

Figs. 4-6 illustrate the importance of a highly flexible edge which is capable of properly conforming to the shape of the bone.

It is noted that the barrier element according to the invention can also be fitted on the side of a bone portion opposite the side where a dental implant is to be attached, so as to allow additional bone tissue to grow on that opposite side, into which the implant may project.

The barrier element according to the invention may also be designed as a blank, which can be rolled up into a tube 4, as shown in Figs. 7-14. Such a tube 4 can be wound around separate parts 5, 6 (Fig. 14) of a bone, so that, firstly, these parts 5 and 6 are positioned and oriented relative to each other and, secondly, a space between these parts 5, 6 where bone can be regenerated is screened off.

In the barrier elements according to Figs. 13 and 14, flexible portions are formed inasmuch as the blanks wound into tubes have strips 7 of decreasing thickness, which, after the winding operation, constitute the ends of the tubes formed. Like the flexible portions 2 of the barrier elements according to Figs. 1-6, the flexible portions 7, when the barrier element is being fitted, can effectively adapt to the shape of the adjacent bone tissue, yielding a good seal between the barrier element and the adjacent bone tissue.

To promote an effective seal of the junction between the barrier element and the bone tissue, the flexible portion 2 may further be shaped as a cupped spring washer. In that case, when the barrier element is being attached, for instance at points indicated by the arrows 9, the flexible edge 2 is pressed against the bone tissue along the outer edge thereof with additional pretension. An improved junction with the bone 3 is then also obtained when the barrier element is attached to a relatively narrow bone portion, as shown in Fig. 6.

With the barrier elements according to Figs. 1-6, too, it is advantageous, with a view to sealing the junction of the barrier element and the bone tissue 3 (see Figs. 4-6), if the wall thickness of the flexible portion decreases starting from the screening portion 1.

Generally, an improved form-retaining ability of the barrier element and an improved seal along the edge thereof are obtained if the barrier element is provided with a sealing lip 8 which is bent over relatively to the screening portion 1. Because the sealing lip 8, as viewed in cross-section, extends, in the transverse direction thereof, at an angle relative to adjacent parts of the screening portion 1, it provides, on the one hand, a stiffening of those adjacent parts of the screening portion 1 and, on the other, it increases the ability of the barrier element to adapt, along its circumferential edge, to the shape of adjacent bone or dental parts of the patient. Accordingly, on the one hand, a reinforcement of the screening is obtained, and, on the other, an improvement of the seal along the edge of the barrier element is obtained.

Barrier elements with such sealing lips 8 are also depicted in Figs. 17, 19 and 20.

In the barrier element shown in Fig. 17, the sealing lip includes an obtuse angle α with adjacent parts of the screening portion 1. This provides the advantage that it is ensured that the lip 8, when the barrier element is being pressed onto a dental element of the patient, is bent away from the screening portion 1 and does not buckle towards the screening portion 1, which would result in less good sealing action.

Fig. 18 shows an example of a dental element 22 having a flank which, in top plan view, comprises a concave portion 21, and a barrier element has been fitted on that flank. Heretofore, the problem encountered in such applications has been that in the area of the concave part, no proper seal between the barrier element and the dental element is obtained.

The sealing lip 8 of the barrier element shown in Fig. 19 is further provided with a channel 19 extending in the longitudinal direction thereof. As a result, an improved seal of concave portions 21 of bone tissue or a dental element is obtained. The channel may for instance be manufactured by injection moulding with inclusion of an insert shaped in conformity with the intended shape of the channel 19 and subsequently removing the insert.

The sealing lip 8 of the barrier element shown in Fig. 20 curls away from the screening portion 1. By designing a barrier element with such a sealing lip, an improved seal along concavities can be obtained as well. A barrier element with a sealing lip 8 as shown in Fig. 20 is moreover easier to manufacture by injection moulding and simpler to sterilize than a barrier element as shown in Fig. 19.

The barrier elements shown in Figs. 15, 16, 18, 19 and 20 comprise a stitching wire 23 which may have been provided by placing it in the die prior to injection moulding and injecting the material of the barrier element around the wire. The wire should be made of material having a lower melting point than the melting point of the material of the barrier element. This can for instance be achieved by using a wire from polyglycolide and using polylactide as material for the barrier element. Thus, the required difference in melting points can be achieved when two biodegradable materials are used.

Fig. 22 shows a die with two die halves 25 and 26, between which a stitching wire 23 is clamped, which extends through a die cavity 27 between the die halves 25 and 26. The die half 25 is provided with protrusions 28 over which the stitching wire is stretched so as to keep the wire spaced from the walls of the die cavity and to effect its being embedded more or less centrally in the barrier element.

The barrier element shown in Fig. 17 is provided with a channel 24 in which a stitching wire can be arranged. The opening of the channel 24 is narrower than the largest width of the channel 24, so that a stitching wire that fits accurately into the channel 24 is retained in the channel 24.

In particular if a barrier element is to be attached to a dental element, it is advantageous if the grooves 20 (Fig. 21) extend approximately parallel to the sealing lip 8 to be arranged against the dental element, because, after implantation, the boundary of the mucous membrane on the side of the dental element generally extends along a line parallel to that sealing lip and growth of the mucous membrane in a direction transverse to that boundary and along the barrier element should be prevented.

In a barrier element according to Figs. 7-9, it is possible to obtain an increased pressure force of the flexible edge 7, and hence an improved seal, if, in the condition where the barrier element has been rolled up into a tube, the diameter of the tube along its ends is smaller than at any portion of the tube located between the ends.

A tubular barrier element of such a shape can be obtained by winding the barrier element about a mandrel 10 comprising two coaxially spaced portions 17 of decreasing diameter, the portions having the largest diameter facing each other. During the winding operation, or subsequently thereto, the barrier element can be heated, allowing it to conform to the shape of the mandrel through plastic deformation. To make such plastic deformation possible, it is sufficient to heat to approx. 70°C in the case where the material used for the barrier element is a polylactic acid.

For the purpose of heating the barrier element during the winding operation, the mandrel may be provided with means for heating the mandrel, such as a heating element or a space for heated liquid.

After cooling, the barrier element can be removed from the mandrel 10 again. It should then be so flexible as to allow it to be bent open so as to be fitted around bone portions, such as the bone portions 5, 6.

A barrier element according to the invention can advantageously be attached by means of fastening means with a shank, which is so shaped that it can be fitted in a matching bore in bone tissue by exerting a force exclusively in the longitudinal direction of the shank. Such fastening means, which are the subject of applicant's Dutch patent application titled "Fastening element for attaching an implant to bone tissue" can be designed with a shank of a particularly small diameter and arranged with minor interspaces, which is advantageous with a view to obtaining a proper junction between the barrier element and the bone tissue. A further advantage of these fastening means is that they can be fitted very rapidly, so that the length of the operation is shortened or, conversely, a relatively large number of fastening means can be applied without presenting any serious problems with regard to the time required for fitting them.

The barrier element shown in Fig. 12 is so shaped that, as the barrier element is being wound onto the mandrel, more layers are formed in the central area, whereas substantially one layer is formed in the area of the ends. This yields an additional reinforcement of the barrier element in the area of the fracture of the bone and the screened space between the bone portions 5 and 6.

When an object of polylactic acid material is being degraded in a human or animal body, the material gradually degrades from the moment of implantation, which manifests itself in the decrease of the molecular weight. When the molecular weight attains a given value, the material becomes soluble and a large proportion of the material of the object disappears within a given period. According to the article by Schakenraad and Dijkstra, mentioned in the introduction to the present specification, the molecular weight at which the material becomes soluble is approx. 5,000 - 10,000 for copolymer of DL lactide and glycolide and approximately 90% of the material of the barrier element disappears within a period of approximately 30-60 days.

During the period in which the material loses the greater part of its mass, a relatively large amount of material is released into the body and the body will react to it. If a barrier element of large dimensions is used, this reaction may be undesirably strong.

The barrier elements according to the exemplary embodiments of the invention shown in Figs. 7-10 each comprise a first portion 11 made from a first resorbable material and a group of second portions 12 made from a second resorbable material (for clarity, only a few second portions are designated by reference numerals). The materials have a structure and composition such that, after implantation, the material of the first portion 11 on average degrades more slowly than does the material of the second portions 12.

As a result, the greater proportion of the material of the second portions 12 is released in a period before the greater part of the material of the first portion 11 is released. Owing to the fact that the release of the greater part of the material of the barrier element is spread over two periods, the amount of material which is released per unit time is considerably smaller than in the case of a similar object made from a single material. Since per unit time smaller amounts of material are released into the body, the body will react less strongly. Further, the strength of the barrier element does not decrease relatively abruptly but in a stepped manner. The strength of the material may thus be adapted to the gradual increase in strength of the bone area to be cured.

Preferably, the structure and the composition of the above-mentioned materials are such that, after implantation, the material of the first portion 11 does not begin to become soluble until substantially all of the material of the further portions 12 has been dissolved or degraded. An overlap of the periods in which the greater part of the two materials disappears is thus avoided. An overlap of the two periods can lead to a temporarily increased supply of material being released from the barrier element, so that a temporarily intensified reaction may occur. However, if the rate at which material is being released is lower at the beginning and at the end of the two periods than in the middle of those periods, the periods may overlap slightly without this leading to a temporarily increased rate of release of material.

A very reliable time difference between periods in which the greater part of the material of each of the two portions 11 and 12 is released, can be obtained if the material of the first portion 11 has a different composition than the material of the second portions 12.

Suitable materials include copolymers of DL lactide and glycolide, the glycolide proportion of the material of the second portion 12 being higher than the glycolide proportion of the material of the first portion 11.

A favorable time interval between the periods in which the greater part of the material of the second portions 12 and the first portion 11, respectively, degrade, can be obtained if the molar percentage of glycolide in the material of the second portion is between 65 and 90% and the molar percentage of glycolide in the material of the first portion is between 40 and 65%.

If a relatively long residence time of the first portion 11 is desired, poly(L-lactide) can advantageously be used for that purpose.

It is also possible, however, to use material of a substantially identical composition for the first and the further portions. A shift of the period in which the greater part of the material of the first and the second portions, respectively, is released from the barrier element can be obtained when the structure of the material of the first portion 11 is more crystalline than the structure of the material of the second portions 12. The use of material of an identical composition for the first and further portions provides the advantage that a single extruder screw will be sufficient for the manufacture thereof.

For a detailed description of process parameters which affect crystallinity, reference is made to a publication by Offergeld, Michaeli and Menges, titled 'The Injection Moulding of Resorbable Implants' in ANTEC '89, pp. 1282-1286.

It is also possible to design the barrier element in such a manner that the portions of different degradation rates gradually merge into each other. To that end, it is for instance possible, when a barrier element is being moulded in a die, for the die to be locally heated and cooled, so that portions of the barrier element cool rapidly and other portions slowly. The slowly cooled portions will exhibit a more amorphous structure and the rapidly cooled portions will exhibit a more crystalline structure.

An even further spread of the periods in which material of the barrier element is released can be achieved if, according to a further exemplary embodiment of the invention (not shown), a third portion of the barrier element is made of a third resorbable material, the material of this third portion having a structure and composition such that, after implantation, it degrades on average more rapidly than does the material of the second portion. Insofar as suitable materials of sufficiently different degradation rates are available, it may be advantageous to further increase the number of portions made of material of mutually different degradation rates.

In the exemplary embodiments of the invention shown in Figs. 7-10, the first portion 11 constitutes a skeleton and the further second portions 12 each fill up a residual space between that skeleton and the outer surface of the barrier element. This provides the advantage that the attack of the portions that are to be degraded first starts immediately after implantation, because these portions are located on the outside of the barrier element. Further, the boundaries between the portions, if present, extend as far as or close to the surface of the barrier element. Through transport of material along these boundaries, accelerated degradation in the area thereof is possible.

The barrier elements according to Figs. 7-10 can each be wound into a tube. The first portion 11 comprises ribs 13 and the second portions 12 fill up the cavities between those ribs. As a result, the barrier element remains closed and maintains a considerable stability and resistance to collapse when the second portions 12 have disappeared. It is important for the barrier element to remain closed in order to keep surrounding tissue outside the space screened by the barrier element, so that the progress of the osteogenesis is not hampered by the penetration of other tissue that grows more rapidly.

The barrier elements according to the exemplary embodiments shown can be plastically deformed after moderate heating. For polylactic acid, heating to approx. 70°C, for instance in a bath of hot water, will generally be sufficient to enable plastic deformation. As a result, the shape of the barrier element can still be adapted during surgical implantation.

Apertures through which the fastening means can be inserted preferably extend through the first portion 11, which has the lowest degradation rate, so that after the disappearance of the second portions 12 of the barrier element, the residues 11 of the barrier element remain fixed in place.

The barrier element according to Figs. 7 and 8 is provided with recesses 14 for centering a drill. These recesses 14 are provided in such a manner that bores starting from those recesses extend through the first portion 11.

The barrier element according to the invention can advantageously be manufactured by introducing a first amount of material into a die, subsequently enlarging the cavity of the die by displacing parts of the die and subsequently introducing a second amount of material into the die. The first portion formed need not be removed from the die, so that the risk of entrapping contaminations between the first portion and the second portion, subsequently injected, is limited to a minimum.

As a result of shrink of the first portion, material of further, second portions may penetrate not only into cavities or residual spaces between the first portion and the enlarged die cavity, but also between the first portion and non-enlarged portions of the die, thus surrounding the first portion. This, however, is not serious because only a thin layer of material is involved, which, moreover, degrades relatively rapidly and disappears.

For the practice of this method, it is advantageous to use an injection moulding machine provided with a die holder and at least two extruder screws for injecting material into one and the same die in that die holder. With this machine, different materials can be introduced into one and the same die in a simple manner and in quick succession.

It is also possible, however, to manufacture a barrier element according to the invention by first forming a first portion in a first die and subsequently placing this in a second die, where second, further portions are formed around the first portion.

A suitable manufacturing method for forming barrier elements of a constant sectional profile, such as the barrier element according to Figs. 9 and 10, is co-extrusion.

Within the concept of the invention, many embodiments other than those shown are conceivable. Thus, it is for instance possible to give the barrier element a simple sandwich structure.

## Claims

1. A bioresorbable barrier element for keeping surrounding tissue outside a space adjacent to bone tissue, so as to obtain osteogenesis, characterized in that it comprises a bending-stiff screening portion (1) formed from synthetic material.

2. A barrier element according to claim 1, characterized in that the screening portion (1) has a wall of solid material.

3. A barrier element according to claim 1 or 2, characterized in that it is constructed as an injection moulded product.

4. A barrier element according to any one of the preceding claims, characterized in that the screening portion is provided with stiffening ribs (18, 19).

5. A barrier element according to claim 4, characterized in that the stiffening ribs (18) are constructed as partitions having a height profile such that, in implanted condition, the partitions rest against a part of a dental element.

6. A barrier element according to any one of the preceding claims, characterized by a texture of grooves (20) located next to each other, of a width and a depth of approximately 100 µm.

7. A barrier element according to any one of the preceding claims, characterized by a central, dish-shaped screening portion (1) and a thin-walled, annular portion (2) extending along the edge of the screening portion (1).

8. A barrier element according to claim 7, characterized in that the flexible portion (2) is shaped as a cupped spring washer.

9. A barrier element according to any one of the preceding claims, characterized in that it is constructed as a blank which can be rolled up into a tube (4).

10. A barrier element according to any one of the preceding claims, characterized by a screening portion (1) and at least one sealing lip (8) along an edge of the screening portion (1).

11. A barrier element according to claim 10, characterized in that the wall thickness of the sealing lip (8) decreases from the screening portion (1).

12. A barrier element according to claim 10 or 11, characterized in that the sealing lip (8), viewed in cross-section, has a bent configuration relative to the adjacent parts of the screening portion (1).

13. A barrier element according to claim 12, characterized in that the sealing lip (8) and said adjacent parts of the screening portion (1) include an obtuse angle.

14. A barrier element according to any one of claims 10-13, characterized in that the sealing lip (8) comprises a channel (19) extending in the longitudinal direction thereof.

15. A barrier element according to any one of claims 10-14, characterized in that the sealing lip (8) curls away from the screening portion (1).

16. A barrier element according to claim 6 and any one of claims 10-15, characterized in that it is designed to be placed against a flank of a dental element (22) and the grooves (20) extend approximately parallel to the sealing lip (8).

17. A barrier element according to claim 9 and any one of claims 10-15, characterized in that, in the condition where it has been rolled up into a tube (4), two flexible portions (7) extend along the ends of the tube.

18. A barrier element according to claim 17, characterized in that, in the condition where it has been rolled up into a tube (4), the diameter of the tube in unloaded condition is smaller along the ends than in any portion of the tube that is located between the ends.

19. A barrier element according to any one of the preceding claims, characterized by bores for fitting fastening means.

20. A barrier element according to any one of the preceding claims, characterized by recesses (14) for centering a perforating element.

21. A barrier element according to any one of the preceding claims, characterized by a composite structure, comprising a first portion (11) made from a first resorbable material and a second portion (12) made from a second resorbable material, said materials having a structure and composition such that, after implantation, the material of the first portion (11) on average degrades more slowly than does the material of the second portion (12).

22. A barrier element according to claim 21, characterized in that the structure and the composition of said materials are such that, after implantation, the material of the first portion (11) begins to become soluble after substantially all of the material of the second portion (12) has been dissolved or degraded.

23. A barrier element according to claim 21 or 22, characterized in that the material of the first portion (11) has a different composition than does the material of the second portion (12).

24. A barrier element according to claim 23, characterized in that both materials are copolymers of DL lactide and glycolide, the glycolide proportion of the material of the second portion being higher than the glycolide proportion of the material of the first portion.

25. A barrier element according to claim 24, characterized in that the molar percentage of glycolide in the material of the second portion is between 65 and 90% and the molar percentage of glycolide in the material of the first portion is between 40 and 65%.

26. A barrier element according to claim 21 or 22, characterized in that the material of the first portion (11) has substantially the same composition as the material of the second portion (12), the structure of the material of the first portion being more crystalline than the structure of the material of the second portion.

27. A barrier element according to any one of claims 21-26, characterized by a third portion having a structure and composition such that, after implantation, the material of that third portion on average degrades more rapidly than does the material of the second portion.

28. A barrier element according to any one of claims 21-27, characterized in that the first portion (11) constitutes a skeleton and the second portion (12) fills up at least a residual space between that skeleton and the outer surface of the barrier element.

29. A barrier element according to claim 19 and any one of claims 21-28, characterized in that the bores for fastening means are provided in said first portion.

30. A barrier element according to claim 20 and any one of claims 21-29, characterized in that the recesses (14) for centering the perforating element are provided in said first portion.

31. An assembly of parts, comprising a barrier element according to any one of the preceding claims and fastening means (15) comprising a head and a shank, the head being provided with a hold-down surface extending at least along a part of its circumferential edge, and the shank being of such shape that it can be secured in a matching bore in bone tissue through the exertion of a force exclusively in the longitudinal direction of the shank.

32. A mandrel for rolling a barrier element according to claim 18 into a tube, characterized by two coaxially spaced portions (17) of decreasing diameter, the portions with the largest diameter facing each other.

33. A mandrel for rolling a barrier element according to claim 9, 17 or 18 into a tube, characterized by means for heating the mandrel.

34. A method for manufacturing a barrier element from resorbable polymeric material, characterized in that a first amount of material is introduced into a die, the cavity of the die is subsequently enlarged by displacing parts of the die and a second amount of material is subsequently introduced into the die.

35. An injection moulding machine for manufacturing a barrier element from resorbable polymer material, characterized by a die holder and at least two extruder screws arranged for injecting material into one and the same die in said die holder.
